# EUROPEAN PATENT APPLICATION

(11) **EP 4 647 502 A1**
(43) Date of publication of application: **12.11.2025**
(21) Application number: 24763218.5
(22) Date of filing: 29.02.2024
(51) Int. Cl.: C12P 13/12, C12N 15/63, C12P 11/00, C12N 9/10

(54) **METHOD FOR DIRECTLY PREPARING L-METHIONINE FROM L-HOMOSERINE**

(30) Priority: 01.03.2023 CN 202310186765
(71) Applicant: Hunan Lier Biotech Co., Ltd., Jia Shan Street Jinshi Hunan 415400 (CN)
(72) Inventor: LAI, Fan, Changde, Hunan 415400 (CN); FAN, Qian, Changde, Hunan 415400 (CN); DAI, Yonghua, Changde, Hunan 415400 (CN); YANG, Pingping, Changde, Hunan 415400 (CN); REN, Jie, Changde, Hunan 415400 (CN)
(74) Representative: Michalski Hüttermann & Partner Patentanwälte mbB
(86) International application number: PCT/CN2024/079268
(87) International publication number: WO 2024/179538

(57) **Abstract**

Provided is a process for preparing L-methionine by enzymatic catalysis reaction of homoserine and methanthiol. The process according to the present disclosure does not comprise a step of generating O-acyl-L-homoserine, and/or O-acyl-L-homoserine is not sued in the process.

## Description

### Technical Field

The present disclosure belongs to the field of biochemical engineering and specifically relates to a process for direct enzymatic preparation of L-methionine using L-homoserine as a substrate.

### Background

Methionine is one of the 20 natural amino acids in proteins and a precursor for the synthesis of S-adenosine methionine, which serves as a donor of methyl group in the human body and is involved in various important metabolic processes. Human cannot synthesize methionine and have to obtain it through (for example) food. Methionine is also a feed additive widely used in livestock, poultry, and fish farming.

Methionine may be prepared through chemical synthesis. However, chemically synthesized methionine is racemic D/L mixed methionine, while D-methionine is difficult to be utilized in living organisms.

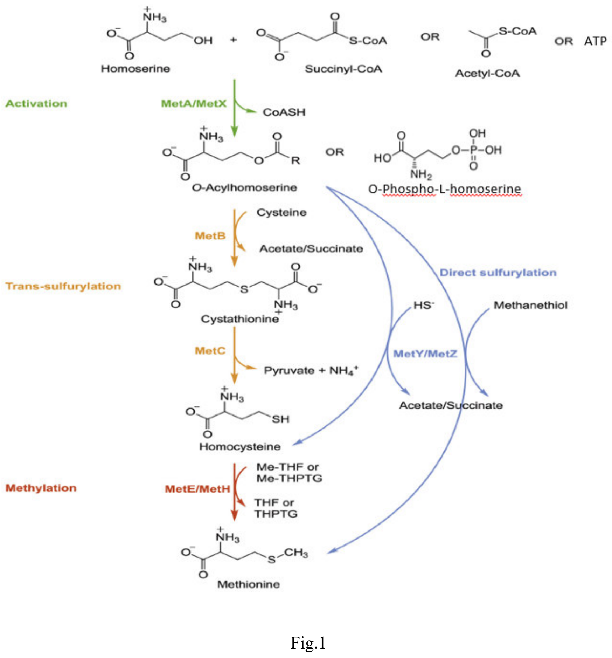

Optically pure L-methionine may also be produced through biosynthesis, achieving high bioavailability. Biosynthesis of methionine is usually conducted by introducing an enzyme involved in the methionine biosynthesis metabolic pathway (see Fig. 1) into a host cell (see e.g., CN 105886449 B, CN 106906176 A, CN 103781913 B and CN 114787369 A). However, biosynthesis requires introduction of a large number of exogenous genes into a host cell, requiring complex genetic engineering modulations. Moreover, due to the complex regulatory network controlling the synthetic metabolic pathway of methionine, it is difficult to achieve a high yield.

A process is also developed to synthesize methionine by sulfurization and methylation of O-acetyl homoserine using methyl terminated thioether, such as dimethyl disulfide, as source of sulfur and methyl, through fermentation with *Corynebacterium glutamicum* or *Escherichia coli* mutant (see, CN 101223280 B). However, in this process, the methanthiol produced by decomposition of thioether has a strong toxic effect on microorganisms, affecting the growth and hindering the synthesis of methionine.

A process for synthesizing L-methionine by catalyzing the reaction of O-succinyl homoserine and sodium methanethiolate using O-succinyl thioltransferase (see CN 113215124 A) and a process for synthesizing L-methionine by catalyzing the reaction of O-acyl homoserine such as O-acetyl homoserine or O-succinyl homoserine with methanthiol using O-acyl homoserine sulfhydrylase (see WO 2008/013432 A1) were also developed.

However, these processes require the production (e.g., through fermentation) of O-acyl homoserine first, which is limited by i) accumulation of O-acyl homoserine; ii) instability of O-acyl homoserine (to acid, base, and heating); iii) inhibition of enzymatic activity by by-product organic acids; and iv) if the pH is adjusted in the reaction process, the production cost, process complexity and product separation difficulty will be increased.

Therefore, there is a need of developing a new process for preparing L-glufosinate, using a stable substrate which is easily obtainable, reducing product inhibition of the reaction, simplifying the process (including production and separation), and reducing costs.

### Summary

Surprisingly, the present inventors found that L-homoserine can react with methanthiol under enzymatic catalysis to produce L-methionine and water (as shown in Formula I).

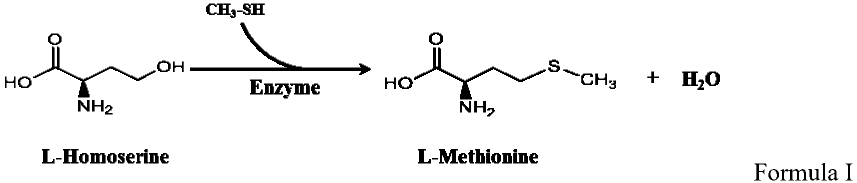

L-homoserine is a compound which is more easily obtainable and stable than O-acyl homoserine, and the reaction of the present disclosure does not produce an organic acid byproduct which inhibits enzymatic activity.

Accordingly, provided is a process for preparing L-methionine, comprising steps of:
a) providing a reaction medium, comprising L-homoserine or a salt, an ester, an amide or an anhydride thereof, methanthiol or a salt thereof, and an enzyme selected from the group consisting of a transferase which transfers an alkyl other than methyl or an aryl, an ammonia lyase, and a lyase which catalyzes the cleavage of a carbon-sulfur bond;
b) incubating the reaction medium to produce a reaction product comprising L-methionine or a salt, an ester, or an amide thereof; and
   optionally,
c) recovering the L-methionine or the salt, the ester, or the amide thereof from the reaction medium.

In some embodiments, the enzyme is selected from the group consisting of enzymes with the EC numbers of EC 2.5.1.-, EC 4.3.1.- and EC 4.4.1.-.

In some embodiments, the reaction medium further comprises phosphopyridoxal or a salt thereof.

In some embodiments, the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

In some embodiments, the enzyme is derived from an organism selected from the group consisting of *Thermus thermophiles, Lysinibacillus sphaericus, Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

In some embodiments, the enzyme is selected from the group consisting of cysteine synthase derived from *Thermus thermophiles* (EC 2.5.1.47 - cysteine synthase), cystathionine γ-synthase derived from *Lysinibacillus sphaericus* (EC 2.5.1.48 - cystathionine gamma-synthase), O-acetyl homoserine aminocarboxypropyltransferase derived from *Clostridioides difficile* (EC 2.5.1.49 - O-acetylhomoserine aminocarboxypropyltransferase), O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis* (EC 2.5.1.51 - beta-pyrazolylalanine synthase), L-mimosine synthase derived from *Leucaena leucocephala* (EC 2.5.1.52 - L-mimosine synthase), O-phosphoserine sulfhydrylase derived from *Aeropyrum pernix* (EC 2.5.1.65 - O-phosphoserine sulfhydrylase), cysteine synthase derived from *Methanosarcina acetivorans* (EC 2.5.1.76 - cysteate synthase), [CysO sulfur-carrier protein]-thiocarboxylate-dependent cysteine synthase derived from *Mycobacterium tuberculosis* (EC 2.5.1.113 - [CysO sulfur-carrier protein]-thiocarboxylate-dependent cysteine synthase), cystathionine β synthase derived from *Bacillus subtilis* (EC 2.5.1.134 - cystathionine beta-synthase), N-(2-amino-2-carboxyethyl)-L-glutamate synthase derived from *Staphylococcus aureus* (EC 2.5.1.140 - N-(2-amino-2-carboxyethyl)-L-glutamate synthase), S-sulfo-L-cysteine synthase derived from *Pseudomonas fluorescens* (EC 2.5.1.144 - S-sulfo-L-cysteine synthase), diaminopropionate ammonia-lyase derived from *Escherichia coli* (EC 4.3.1.15 - Diaminopropionate ammonia-lyase), L-serine ammonia-lyase derived from *Escherichia coli* 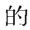 (EC 4.3.1.17 - L-serine ammonia-lyase), D-serine ammonia-lyase derived from *Escherichia coli* (EC 4.3.1.18 - D-serine ammonia-lyase), threonine ammonia-lyase derived from *Candida maltose* (EC 4.3.1.19 - threonine ammonia-lyase), cystathionine γ lyase derived from *Streptomyces phaeochromogenes* (EC 4.4.1.1 - cystathionine gamma-lyase), cystathionine γ lyase derived from *Neurospora crassa* (EC 4.4.1.1 - cystathionine gamma-lyase), homocysteine desulfhydrase derived from *Bacillus subtilis* 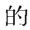 (EC 4.4.1.2 - homocysteine desulfhydrase), L-3-cyanoalanine synthase derived from *Spinacia oleracea* (EC 4.4.1.9 - L-3-cyanoalanine synthase), cysteine lyase derived from *Cyanobacteria bacterium* (EC 4.4.1.10 - cysteine lyase), methionine γ lyase derived from *Pseudomonas putida* (EC 4.4.1.11 - methionine gamma-lyase), cysteine-S-conjugate β lyase derived from *Lactococcus lactis* (EC 4.4.1.13 - cysteine-S-conjugate beta-lyase), D-cysteine desulfhydrase derived from *Escherichia coli* (EC 4.4.1.15 - D-cysteine desulfhydrase), selenocysteine lyase derived from *Escherichia coli* (EC 4.4.1.16 - selenocysteine lyase), L-cysteate sulfo-lyase derived from *Ruegeria pomeroyi* (EC 4.4.1.25 - L-cysteate sulfo-lyase), L-cysteine desulfidase derived from *Methanococcus maripaludis* (EC 4.4.1.28 - L-cysteine desulfidase) and L-cystine β lyase derived from *Camelina sativa* 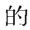 (EC 4.4.1.35 - L-cystine beta-lyase).

In some embodiments, the enzyme comprises an amino acid sequence selected from the group consisting of SEQ ID NO: 1-31.

In some embodiments, the process is a cell-free process.

In some embodiments, the reaction medium in step a) comprises a host cell expressing the enzyme. In some embodiments, the process further comprises a step of culturing the host cell.

In some embodiments, in step b), the reaction medium is incubated at 30-40°C, 32-39°C, 34-38°C, 36-38°C or 37°C.

### Brief Description of the Drawings

Figure 1 shows a schematic diagram of the biosynthetic pathway of methionine (see, Brewster, J. L., et al., Structures and kinetics of Thermotoga maritima MetY reveal new insights into the predominant sulfurylation enzyme of bacterial methionine biosynthesis. The Journal of biological chemistry, 2021, 296, 100797).

### Detailed Description

Provided is a novel process for preparing L-methionine. Unless otherwise stated, the terms used herein have the general understanding of those skilled in the art.

According to reports in the art, 4-hydroxyl activated L-homoserine (such as O-acyl homoserine) can react with methanthiol under enzymatic catalysis to produce L-methionine.

Surprisingly, the present inventors found that L-homoserine can react with methanthiol under enzymatic catalysis to produce L-methionine and water (as shown in Formula I).

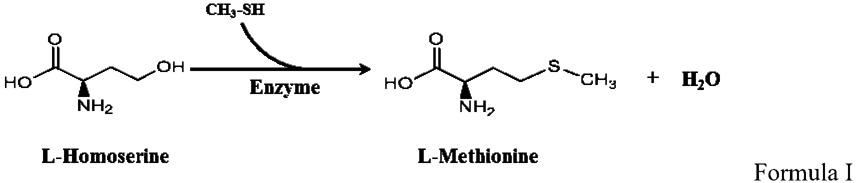

L-homoserine is a compound which is easier to obtain and more stable than O-acyl homoserine, and the reaction of Formula I does not produce an organic acid byproduct which inhibits enzymatic activity.

Accordingly, provided is a process for preparing L-methionine comprising steps of:
a) providing a reaction medium, comprising a L-homoserine donor, a methylthio donor, and an enzyme selected from the group consisting of a transferase which transfers an alkyl other than methyl or an aryl, an ammonia lyase, and a lyase which catalyzes the cleavage of a carbon-sulfur bond;
b) incubating the reaction medium to produce a reaction product comprising L-methionine or a salt, an ester, or an amide thereof; and
   optionally,
c) recovering the L-methionine or a salt, an ester, or an amide thereof from the reaction medium.

### I. Substrate and Product

As used herein, "L-homoserine" refers to L-2-amino-4-hydroxyl-1-acid, with the molecular formula of C₄H₉NO₃. As used herein, a L-homoserine donor comprises but not limited to L-homoserine or a salt, an ester or an amide thereof, as well as an anhydride formed by L-homoserine with L-homoserine or another acid. The L-homoserine donor also comprises a derivative with one or two hydrogens substituted on its amino group. The ester of L-homoserine refers to a compound produced by the reaction of the carboxyl group of L-homoserine with an alcohol or a phenol. The amide of L-homoserine refers to a compound produced by the reaction of the carboxyl group of L-homoserine with an amine compound.

As used herein, the L-homoserine donor does not comprise a derivative where the hydrogen atom of 4-hydroxyl is substituted by acyl, i.e., O-acyl homoserine.

In step b), L-homoserine is the direct substrate of the enzyme. The process according to the present disclosure does not comprise a step of generating O-acyl-L-homoserine, and O-acyl-L-homoserine is not used in the process.

As used herein, "methylthio donor" refers to a compound which can provide methylthio (CH₃S-), comprising but not limited to methanthiol or a salt thereof, as well as methyl terminated thioether or disulfide, such as dimethyl disulfide. In some embodiments, the methylthio donor is methanthiol or a salt thereof, comprising but not limited to sodium methanethiolate and potassium methanethiolate.

### II. Enzyme

As mentioned above, the reaction of the present process is conducted under the catalysis of enzymes, i.e., an enzymatic reaction.

As used herein, the term "enzyme" refers to a protein or peptide which can specifically catalyze or promote a chemical or biochemical reaction. The enzyme according to the present disclosure may be an intact natural protein or polypeptide or a portion thereof with catalytic activity. The enzyme according to the present disclosure may also be a modified variant of a natural protein or polypeptide. The enzyme according to the present disclosure may be isolated from a living organism, or produced by recombination or synthesis. Those skilled in the art can obtain enzymes through conventional methods.

As used herein, the term "peptide" refers to a chain of at least two amino acids connected by peptide bonds. The term "polypeptide" can be used interchangeably with the term "protein" herein, referring to a chain containing ten or more amino acid residues. All peptide and polypeptide chemical formulae or sequences herein are written from left to right, indicating the direction from the amino terminal to the carboxyl terminal.

The term "amino acid" comprises naturally occurring amino acids and non-naturally occurring amino acids in proteins. The single letter and three letter names of naturally occurring amino acids in proteins are commonly used in the art, see Sambrook, et al. (Molecular Cloning: A Laboratory Manual, 2nd, ed. Cold Spring Harbor Laboratory, Cold Spring Harbor Laboratory Press, Cold Spring Harbor, N.Y., 1989).

| Amino Acid | Single letter | Three letter |
|---|---|---|
| Alanine | A | Ala |
| Arginine | R | Arg |
| Asparagine | N | Asn |
| Aspartate | D | Asp |
| Cysteine | C | Cys |
| Glutamine | Q | Gln |
| Glutamate | E | Glu |
| Glycine | G | Gly |
| Histidine | H | His |
| Isoleucine | I | Ile |
| Ieucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

As used herein, the term "modification" refers to any modification of a peptide composed of the polypeptide or its homologous sequence according to the present disclosure, including but not limited to substitution, deletion, insertion, and/or addition of one or more amino acids.

Some enzymes requires the participation of a coenzyme for the catalysis. The term "coenzyme" is a general term for a type of organic cofactors, which are essential factors for enzyme-catalyzed redox reactions, group transfer, and isomerization reactions. They are involved in the function of transferring electrons, atoms, or groups in enzyme-catalyzed reactions.

The present inventors found that the enzyme as used in the present disclosure only uses phosphopyridoxal as a coenzyme. Accordingly, in some embodiments, the reaction medium further comprises phosphopyridoxal or a salt thereof.

In some embodiments, the enzyme is selected from the group consisting of enzymes with the EC numbers of EC 2.5.1.-, EC 4.3.1.- and EC 4.4.1.-.

In some embodiments, the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

In some embodiments, the enzyme is selected from the group consisting of cysteine synthase (EC 2.5.1.47 - cysteine synthase), cystathionine γ-synthase (EC 2.5.1.48 - cystathionine gamma-synthase), O-acetyl homoserine aminocarboxypropyltransferase (EC 2.5.1.49 - O-acetylhomoserine aminocarboxypropyltransferase), βpyrazolylalanine synthase (EC 2.5.1.51 - beta-pyrazolylalanine synthase), L-mimosine synthase (EC 2.5.1.52 - L-mimosine synthase), O-phosphoserine sulfhydrylase (EC 2.5.1.65 - O-phosphoserine sulfhydrylase), cysteine synthase (EC 2.5.1.76 - cysteate synthase), [CysO sulfur-carrier protein]-thiocarboxylate-dependent cysteine synthase (EC 2.5.1.113 - [CysO sulfur-carrier protein]-thiocarboxylate-dependent cysteine synthase), cystathionine β synthase (EC 2.5.1.134 - cystathionine beta-synthase), N-(2-amino-2-carboxyethyl)-L-glutamate synthase (EC 2.5.1.140 - N-(2-amino-2-carboxyethyl)-L-glutamate synthase), S-sulfo-L-cysteine synthase (EC 2.5.1.144 - S-sulfo-L-cysteine synthase), diaminopropionate ammonia-lyase (EC 4.3.1.15 - Diaminopropionate ammonia-lyase), L-serine ammonia-lyase (EC 4.3.1.17 - L-serine ammonia-lyase), D-serine ammonia-lyase (EC 4.3.1.18 - D-serine ammonia-lyase), threonine ammonia-lyase (EC 4.3.1.19 - threonine ammonia-lyase), cystathionine γ lyase (EC 4.4.1.1 - cystathionine gamma-lyase), homocysteine desulfhydrase (EC 4.4.1.2 - homocysteine desulfhydrase), L-3-cyanoalanine synthase (EC 4.4.1.9 - L-3-cyanoalanine synthase), cysteine lyase (EC 4.4.1.10 - cysteine lyase), methionine γ lyase (EC 4.4.1.11 - methionine gamma-lyase), cysteine-S-conjugate β lyase (EC 4.4.1.13 - cysteine-S-conjugate beta-lyase), D-cysteine desulfhydrase (EC 4.4.1.15 - D-cysteine desulfhydrase), selenocysteine lyase (EC 4.4.1.16 - selenocysteine lyase), L-cysteate sulfo-lyase (EC 4.4.1.25 - L-cysteate sulfo-lyase), L-cysteine desulfidase (EC 4.4.1.28 - L-cysteine desulfidase) and L-cystine β lyase (EC 4.4.1.35 - L-cystine beta-lyase).

The enzyme according to the present disclosure may be derived from any suitable organism, including prokaryotes and eukaryotes, for example but not limited to archaea, actinomycetes, bacteria, fungi, animals, plants, algae, and yeast. In some embodiments, the enzyme is derived from an organism selected from the group consisting of *Thermus thermophiles, Lysinibacillus sphaericus), Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

In some embodiments, the enzyme is selected from the group consisting of cysteine synthase derived from *Thermus thermophiles* (EC 2.5.1.47 - cysteine synthase), cystathionine γ-synthase derived from *Lysinibacillus sphaericus* (EC 2.5.1.48 - cystathionine gamma-synthase), O-acetyl homoserine aminocarboxypropyltransferase derived from *Clostridioides difficile* (EC 2.5.1.49 - O-acetylhomoserine aminocarboxypropyltransferase), O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis* (EC 2.5.1.51 - beta-pyrazolylalanine synthase), L-mimosine synthase derived from *Leucaena leucocephala* (EC 2.5.1.52 - L-mimosine synthase), O-phosphoserine sulfhydrylase derived from *Aeropyrum pernix* (EC 2.5.1.65 - O-phosphoserine sulfhydrylase), cysteine synthase derived from *Methanosarcina acetivorans* (EC 2.5.1.76 - cysteate synthase), [CysO sulfur-carrier protein]-thiocarboxylate-dependent cysteine synthase derived from *Mycobacterium tuberculosis* (EC 2.5.1.113 - [CysO sulfur-carrier protein]-thiocarboxylate-dependent cysteine synthase), cystathionine β synthase derived from *Bacillus subtilis* (EC 2.5.1.134 - cystathionine beta-synthase), N-(2-amino-2-carboxyethyl)-L-glutamate synthase derived from *Staphylococcus aureus* (EC 2.5.1.140 - N-(2-amino-2-carboxyethyl)-L-glutamate synthase), S-sulfo-L-cysteine synthase derived from *Pseudomonas fluorescens* (EC 2.5.1.144 - S-sulfo-L-cysteine synthase), diaminopropionate ammonia-lyase derived from *Escherichia coli* (EC 4.3.1.15 - Diaminopropionate ammonia-lyase), L-serine ammonia-lyase derived from *Escherichia coli* (EC 4.3.1.17 - L-serine ammonia-lyase), D-serine ammonia-lyase derived from *Escherichia coli* (EC 4.3.1.18 - D-serine ammonia-lyase), threonine ammonia-lyase derived from *Candida maltose* (EC 4.3.1.19 - threonine ammonia-lyase), cystathionine γ lyase derived from *Streptomyces phaeochromogenes* 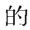 (EC 4.4.1.1 - cystathionine gamma-lyase), cystathionine γ lyase derived from *Neurospora crassa* (EC 4.4.1.1 - cystathionine gamma-lyase), homocysteine desulfhydrase derived from *Bacillus subtilis* (EC 4.4.1.2 - homocysteine desulfhydrase), L-3-cyanoalanine synthase derived from *Spinacia oleracea* (EC 4.4.1.9 - L-3-cyanoalanine synthase), cysteine lyase derived from *Cyanobacteria bacterium* (EC 4.4.1.10 - cysteine lyase), methionine γ lyase derived from *Pseudomonas putida* (EC 4.4.1.11 - methionine gamma-lyase), cysteine-S-conjugate β lyase derived from *Lactococcus lactis* (EC 4.4.1.13 - cysteine-S-conjugate beta-lyase), D-cysteine desulfhydrase derived from *Escherichia coli* (EC 4.4.1.15 - D-cysteine desulfhydrase), selenocysteine lyase derived from *Escherichia coli* (EC 4.4.1.16 - selenocysteine lyase), L-cysteate sulfo-lyase derived from *Ruegeria pomeroyi* (EC 4.4.1.25 - L-cysteate sulfo-lyase), L-cysteine desulfidase derived from *Methanococcus maripaludis* (EC 4.4.1.28 - L-cysteine desulfidase) and L-cystine β lyase derived from *Camelina sativa* (EC 4.4.1.35 - L-cystine beta-lyase). In some embodiments, the enzyme is O-acetyl homoserine aminocarboxypropyltransferase derived from *Clostridium novyi.*

In some embodiments, the enzyme comprises an amino acid sequence selected from the group consisting of SEQ ID NO: 1-31. In some embodiments, the enzyme is a modified variant.

As used herein, the term "variant" refers to a peptide or nucleic acid which has a certain sequence identity compared to the amino acid or nucleotide sequence of a given peptide or nucleic acid.

For the present disclosure, to determine the percentage of identity between two amino acid sequences or two nucleic acid sequences, sequences are aligned for optimal comparison (for example, a gap may be introduced in the first amino acid or nucleic acid sequence for optimal alignment with the second amino acid or nucleic acid sequence). Then the amino acid residues or nucleotides at the corresponding amino acid or nucleotide positions are compared. When the position in the first sequence is occupied by the same amino acid residue or nucleotide in the corresponding position in the second sequence, these molecules are identical at that position. The identity percentage between two sequences is a function of the number of identical positions shared by the sequence (i.e., identity percentage = number of identical positions / total number of positions (i.e., overlapping positions) × 100). Preferably, these two sequences are of the same length.

Those skilled in the art know that various computer programs may be used to determine the identity between two sequences.

"Amino acid identity percentage" or "amino acid sequence identity percentage" refers to comparison of amino acids of two polypeptides, and when the optimal comparison is made, the two polypeptides have approximately the specified percentage of identical amino acids. For example, "95% amino acid identity" means that two polypeptides have 95% identical amino acids when the two polypeptides are compared for amino acids at an optimal alignment.

In some embodiments, the enzyme comprises an amino acid sequence having at least 65% or 70%, preferably at least 75% or 80%, more preferably at least 85% or 90%, particularly preferably at least 94%, 95%, 96%, 97%, 98% or 99%, most preferably at least 99.1%, 99.2%, 99.3%, 99.4%, 99.5%, 99.6%, 99.7%, 99.8% or 99.9% sequence identity with an amino acid sequence selected from the group consisting of SEQ ID NO: 1-31, and the enzyme has the activity of catalyzing the reaction of Formula I.

In some embodiments, the amino acid sequence of the enzyme differs from the amino acid sequence selected from the group consisting of SEQ ID NO: 1-31 in having a substitution, deletion, insertion, and/or addition of one or more amino acids, and the enzyme has the activity of catalyzing the reaction of Formula I. In some embodiments, the amino acid sequence of the enzyme differs from the amino acid sequence selected from the group consisting of SEQ ID NO: 1-31 in having a conserved substitution of one or more amino acids, and the enzyme has the activity of catalyzing the reaction of Formula I. In some embodiments, the amino acid sequence of the enzyme differs from the amino acid sequence selected from the group consisting of SEQ ID NO: 1-31 in having an insertion or a deletion of one or more amino acids, and the enzyme has the activity of catalyzing the reaction of Formula I.

The term "conserved substitution", also known as substitution with "homologous" amino acid residue, refers to the substitution where an amino acid residue is replaced with another amino acid residue having a similar side chain, such as amino acids with basic side chain (such as lysine, arginine and histidine), amino acids with acidic side chain (such as aspartate, glutamate), amino acids with uncharged polar side chain (such as glycine, asparagine, glutamine, serine, threonine, tyrosine, cysteine), amino acids with nonpolar side chain (such as alanine, valine, leucine, isoleucine, proline, phenylalanine, methionine, tryptophan), amino acids with β-branched side chain (such as threonine, valine, isoleucine) and amino acids with aromatic side chain (such as tyrosine, phenylalanine, tryptophan, histidine).

The conserved amino acid substitution usually has the minimal influence on the activity of the resulted protein. Such substitution is described below. The conserved substitution is the replacement of an amino acid with another amino acid having similar size, hydrophobicity, charge, polarity, spatial characteristics, aromaticity, or the like. When it is desired to finely regulate the properties of a protein, the substitution is usually conserved.

As used herein, "homologous" amino acid residues refer to amino acid residues with similar chemical properties, which involve hydrophobicity, charge, polarity, spatial characteristics, aromatic characteristics, or the like. Examples of amino acids which are homologous to each other comprise positively charged lysine, arginine, histidine; negatively charged glutamate, aspartate; hydrophobic glycine, alanine, valine, leucine, isoleucine, proline, phenylalanine; polar serine, threonine, cysteine, methionine, tryptophan, tyrosine, asparagine, glutamine; aromatic phenylalanine, tyrosine, tryptophan; serine and threonine with chemically similar side chain groups, or glutamine and asparagine, or leucine and isoleucine.

Examples of conservative amino acid substitutions in proteins include: Ser for Ala, Lys for Arg, Gln or His for Asn, Glu for Asp, Ser for Cys, Asn for Gln, Asp for Glu, Pro for Gly, Asn or Gln for His, Leu or Val for Ile, Ile or Val for Leu, Arg or Gln for Lys, Leu or Ile for Met, Met, Leu or Tyr for Phe, Thr for Ser, Ser for Thr, Tyr for Trp, Trp or Phe for Tyr, and Ile or Leu for Val.

### III. Preparation conditions

The enzyme according to the present disclosure can be provided in the form of an isolated polypeptide.

In some embodiments, the process is a cell-free process, that is, the reaction medium in step a) comprises an isolated enzyme. The enzyme may be produced by recombination or synthesis. In some embodiments, the reaction medium is an aqueous medium, preferably an aqueous buffer, for example a phosphate buffer, such as PBS.

In some embodiments, the reaction medium in step a) comprise a host cell expressing the enzyme. In some embodiments, the process according to the present disclosure further comprises a step of culturing the host cell. In some embodiments, the reaction medium comprises a culture medium for culturing the host cell. In some embodiments, the reaction medium is an aqueous medium, preferably an aqueous buffer solution, for example a phosphate buffer solution, such as PBS.

In some embodiments, the host cell comprises an expression vector encoding the enzyme. In some embodiments, the host cell is *Escherichia coli,* for example *Escherichia coli* BL21(DE3). In some embodiments, the expression vector is a prokaryotic expression vector, for example pET-29b(+).

In some embodiments, in step b), the reaction medium is incubated at 30-40°C, 32-39°C, 34-38°C, 36-38°C or 37°C.

In some embodiments, the reaction medium has a PH of about 5-about 8, preferably about 6 - about 7.8, for example 7.5.

### Examples

Through the following examples, those skilled in the art will understand the present disclosure more clearly. It should be noted that the examples are provided for illustrative purpose only rather than limitation to the scope of the present disclosure.

### Example 1. Materials and methods

Unless otherwise stated, the experimental methods used herein are conventional, and specific gene cloning procedures can be found in Sambrook et al., 1989 above.
i) Reagents:
   - Isopropyl-β-D-thiogalactoside (IPTG) was purchased from Beyotime Biotechnology Co., Ltd.
   - L-homoserine was purchased from Jiangsu Aikon Biomedical Research and Development Co., Ltd.
   - L-methionine was purchased from Merck Sigma-Aldrich;
   - Sodium methanethiolate, phosphopyridoxal (PLP), chromatographic grade disodium hydrogen phosphate, sodium tetraborate, methanol, and acetonitrile were purchased from Shanghai Aladdin Biochemical Technology Co., Ltd.
   - Sodium dihydrogen phosphate, sodium hydroxide, and Triton-X100 were purchased from Sangon Biotechnology (Shanghai) Co., Ltd.
   - OPA reagent was purchased from Agilent Technology (China) Co., Ltd
ii) Vectors and strains:
   - The expression vector used in the example was pET-29b(+), purchased from Sangon Biotechnology (Shanghai) Co., Ltd.
   - The host cell used in the example was *Escherichia coli* BL21(DE3), purchased from Tiangen Biochemical Technology (Beijing) Co., Ltd.
iii) Gene clone

The nucleic acid sequences encoding each enzyme in Table 1 were provided to Sangon Biotechnology (Shanghai) Co., Ltd. for synthesis, obtaining pET29b(+) expression vectors containing the nucleic acid sequences encoding each enzyme.

**Table 1**

| Enzyme | EC No. | Species | NCBI-Protein ID | Amino Acid Sequence |
|---|---|---|---|---|
| cysteine synthase | EC 2.5.1.47 | *Thermus thermophilus* | BAD70170 | SEQ ID NO: 1 |
| cystathionine γ-synthase | EC 2.5.1.48 | *Lysinibacillus sphaericus* | ACA40889 | SEQ ID NO: 2 |
| O-acetyl homoserine aminocarboxypropyltransferase | EC 2.5.1.49 | *Clostridioides difficile* | APU51341 | SEQ ID NO: 3 |
| O-acetyl homoserine aminocarboxypropyltransferase | EC 2.5.1.49 | *Clostridium novyi* | WP_01172130 1 | SEQ ID NO: 4 |
| O-acetyl homoserine aminocarboxypropyltransferase | EC 2.5.1.49 | *Geobacillus stearothermophilus* | ALA70660 | SEQ ID NO: 5 |
| O-acetyl homoserine aminocarboxypropyltransferase | EC 2.5.1.49 | *Thermotoga maritima* | WP_00408071 0 | SEQ ID NO: 6 |
| O-acetyl homoserine aminocarboxypropyltransferase | EC 2.5.1.49 | *Pseudomonas aeruginosa* | NP_253712 | SEQ ID NO: 7 |
| β pyrazolylalanine synthase | EC 2.5.1.51 | *Nonlabens dokdonensis* | BAA05965 | SEQ ID NO: 8 |
| L-mimosine synthase | EC 2.5.1.52 | *Leucaena leucocephala* | AHG97874 | SEQ ID NO: 9 |
| O-phosphoserine sulfhydrylase | EC 2.5.1.65 | *Aeropyrum pernix* | BAA80586 | SEQ ID NO: 10 |
| cysteine synthase | EC 2.5.1.76 | *Methanosarcina acetivorans* | AAM06667 | SEQ ID NO: 11 |
| [CysO sulfur-carrier protein]-thiocarboxylate-depe ndent cysteine synthase | EC 2.5.1.113 | *Mycobacterium tuberculosis* | NP_215852 | SEQ ID NO: 12 |
| cystathionine β synthase | EC 2.5.1.134 | *Bacillus subtilis* | NP_390604 | SEQ ID NO: 13 |
| N-(2-amino-2-carboxyethyl)-L-glutamate synthase | EC 2.5.1.140 | *Staphylococcus aureus* | BAF66332 | SEQ ID NO: 14 |
| S-sulfo-L-cysteine synthase | EC 2.5.1.144 | *Pseudomonas fluorescens* | AIG05382 | SEQ ID NO: 15 |
| diaminopropionate ammonia-lyase | EC 4.3.1.15 | *Escherichia coli* | BAE76937 | SEQ ID NO: 16 |
| L-serine ammonia-lyase | EC 4.3.1.17 | *Escherichia coli* | BAA15621 | SEQ ID NO: 17 |
| D-serine ammonia-lyase | EC 4.3.1.18 | *Escherichia coli* | BAA16229 | SEQ ID NO: 18 |
| threonine ammonia-lyase | EC 4.3.1.19 | *Candida maltosa* | EMG50701 | SEQ ID NO: 19 |
| cystathionine γ lyase | EC 4.4.1.1 | *Streptomyces phaeochromogenes* | WP_26676142 6 | SEQ ID NO: 20 |
| cystathionine γ lyase | EC 4.4.1.1 | *Neurospora crassa* | AF401238_1 | SEQ ID NO: 21 |
| homocysteine desulfhydrase | EC 4.4.1.2 | *Bacillus subtilis* | NP_390603 | SEQ ID NO: 22 |
| L-3-cyanoalanine synthase | EC 4.4.1.9 | *Spinacia oleracea* | BAA07177.1 | SEQ ID NO: 23 |
| cysteine lyase | EC 4.4.1.10 | *Cyanobacteria bacterium* | HAA27123 | SEQ ID NO: 24 |
| methionine γ lyase | EC 4.4.1.11 | *Pseudomonas putida* | AAN66932 | SEQ ID NO: 25 |
| cysteine-S-conjugate β lyase | EC 4.4.1.13 | *Lactococcus lactis* | ABJ72384 | SEQ ID NO: 26 |
| D-cysteine desulfhydrase | EC 4.4.1.15 | *Escherichia coli* | BAA15739 | SEQ ID NO: 27 |
| selenocysteine lyase | EC 4.4.1.16 | *Escherichia coli* | BAA15457 | SEQ ID NO: 28 |
| L-cysteate sulfo-lyase | EC 4.4.1.25 | *Ruegeria pomeroyi* | AAV97294 | SEQ ID NO: 29 |
| L-cysteine desulfidase | EC 4.4.1.28 | *Methanococcus maripaludis* | CAF31024 | SEQ ID NO: 30 |
| L-cystine β lyase | EC 4.4.1.35 | *Camelina sativa* | XP_010509309 | SEQ ID NO: 31 |

### iv) Preparation of recombinant host cells

The expression vectors were transformed into *Escherichia coli* BL21(DE3) competent cells, respectively, which were spread om LB agar medium (containing 50mg/L kanamycin), incubated overnight at 37°C. Then single colonies were selected and cultured in LB broth (containing 50mg/L kanamycin) and subjected to sequencing to verify the correctness of the synthesized sequences. The verified clones were stored at -80°C for subsequent experiments.

### v) Protein expression and preparation of whole cell catalysts:

The stored clones were activated on LB agar medium. Then, the single colonies were inoculated in LB broth c(containing 50mg/L kanamycin) and incubated at 37°C for 12 h with shaking. 400 µL of culture was transferred into 20 mL of fresh LB liquid medium (containing 50mg/L kanamycin) and incubated at 37°C with shaking until the OD600 reached about 0.6. IPTG (final concentration of 0.4mM) was added and incubation was conducted at 25°C for 16 h to induce protein expression.

After incubation, the cultures were centrifuged at 4000g for 10 min at 4°C, the supernatants were discarded, and the *Escherichia coli* cells were collected. The collected *Escherichia coli* cells were resuspended in precooled 15 mL of 50 mM PBS, pH 7.0 to give *Escherichia coli* suspension containing the recombinant enzyme as whole cell catalysts.

### vi) Preparation of enzymes

7.5 mL of *Escherichia coli* suspension prepared in v) was taken and the *Escherichia coli* cells were sonicated at 4°C. The cell fragmentation solutions were centrifuged at 4°C at 6000g for 15 min to remove the precipitates, so as to obtain the resultant supernatants as crude enzyme solutions containing the recombinant enzymes. The crude enzyme solutions were purified using AKTA protein purification system to obtain the purified enzymes.

### vii) Catalyzing reaction with purified enzymes

Sodium methanethiolate and PLP were added to a solution of L-homoserine in PBS (100mM), and the solution was adjusted to pH 7.5 with phosphoric acid. In the solution, the final concentration of L-homoserine was 169.7mM, the final concentration of sodium methanethiolate was 419.74mM, and the final concentration of PLP was 0.2mM. Purified enzyme as prepared in vi) was added to the above solution and the final concentration was 8mg/mL. At 37°C, the system was shaken continuously on an oscillator (220rpm) for 12 h. The sample was taken for detecting the yield of L-methionine using OPA pre-column derivatization high performance liquid chromatography to determine the catalytic activity of the purified enzyme.

### viii) Whole cell catalytic reaction

Sodium methanethiolate, PLP, and Triton X-100 were added to a solution of L-homoserine in PBS (100mM) and the solution was adjusted to pH 7.5 with phosphoric acid. In the solution, the final concentration of L-homoserine was 169.7mM, the final concentration of sodium methanethiolate was 419.74mM, the final concentration of PLP was 0.2mM, and the final concentration of Triton X-100 was 0.2%. Cell suspensions prepared in v) were added to the above solutions respectively, and the final concentrations of the whole cells were 25 OD₆₀₀. At 37°C, the system was shaken continuously on an oscillator (220rpm) for 12 h. The sample was taken for detecting the yield of L-methionine using OPA pre-column derivatization high performance liquid chromatography to determine the catalytic activity of the whole cells.

### Example 2. Preparation and detection of MetY (CnMetY) from Clostridium novvi

The clone expressing CnMetY (SEQ ID NO: 4) was activated, purified enzyme was prepared, and the amount of L-methionine produced by catalysis of the purified enzyme was determined according to the procedures in Example 1. Upon detection, 12.13g/L of L-methionine was produced by the catalysis of purified enzyme CnMetY.

### Example 3. Preparation and detection of whole cells containing sulfhydrylase (MetY) and cystathionine γ lyase (CGL)

*Escherichia coli* clones expressing CnMetY(SEQ ID NO: 4), CdMetY(SEQ ID NO: 3), GsMetY(SEQ ID NO: 5), TmMetY(SEQ ID NO: 6), PaMetY(SEQ ID NO: 7), NaCGL(SEQ ID NO: 21), SpCGL(SEQ ID NO: 20) were activated, whole cells were prepared, and the amounts of L-methionine produced by catalysis of the whole cells were detected according to the procedures in Example 1. The determined amounts of L-methionine produced by the catalysis of the whole cells were shown in Table 2.

**Table 2**

| Whole cell | SEQ ID NO: | L-methionine yield (g/L) |
|---|---|---|
| CdMetY | 3 | 0.51 |
| CnMetY | 4 | 13.81 |
| GsMetY | 5 | 0.47 |
| TmMetY | 6 | 1.07 |
| PaMetY | 7 | 0.11 |
| NaCGL | 21 | 0.17 |
| SpCGL | 20 | 0.39 |

### Example 4. Preparation and detection of whole cells containing enzymes other than sulfhydrylase (MetY) and cystathionine γ lyase (CGL) in Table 1

*Escherichia coli* clones expressing enzymes other than sulfhydrylase (MetY) and cystathionine γ lyase (CGL) in Table 1 were activated, whole cells were prepared according to the procedures in Example 1, and the amounts of L-methionine produced by catalysis of the whole cells were detected. The catalytic performances of enzymes were evaluated according to the amounts of L-methionine produced. The catalyzing ability of enzymes were shown in Table 3 and presented by *, more * indicating stronger catalyzing ability.

**Table 3**

| Enzyme | EC No. | Species | Amino Acid Sequence | Catalytic Performance |
|---|---|---|---|---|
| cysteine synthase | EC 2.5.1.47 | *Thermus thermophilus* | SEQ ID NO: 1 | ★ |
| cystathionine γ-synthase | EC 2.5.1.48 | *Lysinibacillus sphaericus* | SEQ ID NO: 2 | ★★★ |
| βpyrazolylalanine synthase | EC 2.5.1.51 | *Nonlabens dokdonensis* | SEQ ID NO: 8 | ★ |
| L-mimosine synthase | EC 2.5.1.52 | *Leucaena leucocephala* | SEQ ID NO: 9 | ★★ |
| O-phosphoserine sulfhydrylase | EC 2.5.1.65 | *Aeropyrum pernix* | SEQ ID NO: 10 | ★ |
| cysteine synthase | EC 2.5.1.76 | *Methanosarcina acetivorans* | SEQ ID NO: 11 | ★★ |
| [CysO sulfur-carrier protein]-thiocarboxylate-dependent cysteine synthase | EC 2.5.1.113 | *Mycobacterium tuberculosis* | SEQ ID NO: 12 | ★ |
| cystathionine β synthase | EC 2.5.1.134 | *Bacillus subtilis* | SEQ ID NO: 13 | ★ |
| N-(2-amino-2-carboxyethyl)-L-gluta mate synthase | EC 2.5.1.140 | *Staphylococcus aureus* | SEQ ID NO: 14 | ★ |
| S-sulfo-L-cysteine synthase | EC 2.5.1.144 | *Pseudomonas fluorescens* | SEQ ID NO: 15 | ★ |
| diaminopropionate ammonia-lyase | EC 4.3.1.15 | *Escherichia coli* | SEQ ID NO: 16 | ★★ |
| L-serine ammonia-lyase | EC 4.3.1.17 | *Escherichia coli* | SEQ ID NO: 17 | ★★ |
| D-serine ammonia-lyase | EC 4.3.1.18 | *Escherichia coli* | SEQ ID NO: 18 | ★★ |
| threonine ammonia-lyase | EC 4.3.1.19 | *Candida maltosa* | SEQ ID NO: 19 | ★ |
| homocysteine desulfhydrase | EC 4.4.1.2 | *Bacillus subtilis* | SEQ ID NO: 22 | ★★★★ |
| L-3-cyanoalanine synthase | EC 4.4.1.9 | *Spinacia oleracea* | SEQ ID NO: 23 | ★ |
| cysteine lyase | EC 4.4.1.10 | *Cyanobacteria bacterium* | SEQ ID NO: 24 | ★ |
| methionine γ lyase | EC 4.4.1.11 | *Pseudomonas putida* | SEQ ID NO: 25 | ★★★★ |
| cysteine-S-conjugate β lyase | EC 4.4.1.13 | *Lactococcus lactis* | SEQ ID NO: 26 | ★★★ |
| D-cysteine desulfhydrase | EC 4.4.1.15 | *Escherichia coli* | SEQ ID NO: 27 | ★ |
| selenocysteine lyase | EC 4.4.1.16 | *Escherichia coli* | SEQ ID NO: 28 | ★ |
| L-cysteate sulfo-lyase | EC 4.4.1.25 | *Ruegeria pomeroyi* | SEQ ID NO: 29 | ★ |
| L-cysteine desulfidase | EC 4.4.1.28 | *Methanococcus maripaludis* | SEQ ID NO: 30 | ★ |
| L-cystine β lyase | EC 4.4.1.35 | *Camelina sativa* | SEQ ID NO: 31 | ★ |

### Sequences

SEQ ID NO: 1 
MRVEGAIGKTPVVRLAKVVEPDMAEVWVKLEGLNPGGSIKDRPAWYMIKDAEE
RGILRPGSGQVIVEPTSGNTGIGLAMIAASRGYRLILTMPAQMSEERKRVLKAFGA
ELVLTDPERRMLAAREEALRLKEELGAFMPDQFKNPANVRAHYETTGPELYEALE
GRIDAFVYGSGTGGTITGVGRYLKERIPHVKVIAVEPARSNVLSGGKMGQHGFQG
MGPGFIPENLDLSLLDGVIQVWEEDAFPLARRLAREEGLFLGMSSGGIVWAALQV
ARELGPGKRVACISPDGGWKYLSTPLYAEP
SEQ ID NO: 2 
MTLNRSIETKLVQLGNLSDPTTGAVSPPIHLSTAYKHAGIGESTGFDYTRTKNPTRA
LLEAGFADLEGGDMGFACSSGMAAIQLVLSIFKPGDELVVPDDLYGGTYRLFNFFQ
ETYNIKPVYSKFESVEQVEALINEHTRALFIETPTNPLMQEFDLQVYAELAHKHGA
LLIVDNTFYTPYFQRPIDLGADIVLHSATKYIGGHNDVLAGIVVAKGTELAERIGFI
HNGSGMVLGAMDSWLLIRGLKTMHLRLKQHDANAKAIAAYLEEEALVTDVLYT
GKGGMLSFRLQKPEWIDPFLRNLKLITFAESLGGVESFITYPATQTHADMPYEERV
ERGVCDRLLRFSVGIEEAEDLIADLRQVFDILRKEA
SEQ ID NO: 3 
MDNKETICVQGNYKPGNGEPRVLPLYQSTTFKYSSIDQLAELFDLKVDGHIYSRIS
NPTIQAFEEKISLLEGGVSSVAVSSGQSANMLAVLNICKSGDSILCSSKVYGGTFNL
LGPSLKKFGIDLISFDLDSSEDEIVELAKENTKVVFAETLANPTLEVIDFEKIANVAK
RINVPFIVDNSLASPVLCNPLKYGANIVTHSTTKYLDGHASSVGGIIVDGGNFNWD
NGKFPELVEPDPTYHGISYTQKFGNAAYATKARVQLLRDYGNCLSPFNAYLTNLN
VETLHLRMERHSENALKIARFLEKHENVDWINYPGLEDNKYYENAKKYLSRGCS
GVLSFGVRGGLENAKKFVEKLQIASLVTHVSDVRTCVIHPASTTHRQLTEEQLIAS
GVLPSLIRLSVGIENVEDLIADLNQALNF
SEQ ID NO: 4 
MNNWKKGTICIQGGYTPKSGEPRVLPIYQSTTYKYNDPDEVAALFDLKAEGHMY
SRISNPTVAAFEEKIAALEGGVGALAVASGQSASTLSILNVCKSGEHVVAASTLYGG
TYSLFSTTLKKFGIDVTFVDPEATEEEILKNCRENTKAVFGETIGNPGLNVLDFEKF
SNVAKKIKAPFIVDNTIATPYLCNPLKLGANVVVHSATKYIDGHATTVGGVIIDGG
NFDWNNGRFPDFTEPDPSYHGIKYTETFGDSAYIVKARVQLLRDLGVCTSPFNAFL
FSLGLETLHLRMERHSDNAIKLGRFLEKHSKVSWVSYPLLESHSTYETAKKYLPK
GASGILTFGVKGGVEAGKQFIRNLKLAALVVHLGDARTSVLHPASTTHRQLTEEEQ
LSAGVTPDLIRVSVGIEDIEDIIEDFDQALNNIK
SEQ ID NO: 5 
MSNEQTFRPETLAIHAGQKPDAETGARAVPIYQTSSYVFRDSEHAANLFGLKEEGF
IYTRIMNPTNDVFEKRIAALEGGIGALALSSGQAAVFYSIINIASAGDEIVSSSSIYGG
TYNLFAHTLRKFGITVKFVDPSDPENFERAITDKTKALFAETIGNPKNDVLDIEAVA
DIAHRHAIPLIVDNTVASPYLLRPIEFGADIVVHSATKFIGGHGNSIGGVIVDSGKFD
WKGSGKFPEFTEPDPSYHGLVYVDAVGEAAYITKARIQLLRDLGAALSPFNAFLLL
QGLETLHLRMQRHSENALAVAKFLEEEEAVESVNYPGLPSHPSHELAKKYLPNGQ
GAIVTFEIKGGVEAGKKLIDSVKLFSHLANIGDSKSLIIHPASTTHEQLSPDEQLSAG
VTPGLVRLSVGTEAIDDILDDLRQAIRQSQTVGVK
SEQ ID NO: 6 
MDWKKYGYNTRALHAGYEPPEQATGSRAVPIYQTTSYVFRDSDHAARLFALEEP
GFIYTRIGNPTVSVLEERIAALEEGVGALAVASGQAAITYAILNIAGPGDEIVSGSAL
YGGTYNLFRHTLYKKSGIIVKFVDETDPKNIEEAITEKTKAVYLETIGNPGLTVPDF
EAIAEIAHRHGVPLIVDNTVAPYIFRPFEHGADIVVYSATKFIGGHGTSIGGLIVDSG
KFDWTNGKFPELVEPDPSYHGVSYVETFKEAAYIAKCRTQLLRDLGSCMSPFNAF
LFILGLETLSLRMKKHCENALKIVEFLKSHPAVSWVNYPIAEGNKTRENALKYLKE
GYGAIVTFGVKGGKEAGKKFIDSLTLISHLANIGDARTLAIHPASTTHQQLTEEEQL
 KTGVTPDMIRLSVGIEDVEDIIADLDQALRKSQEG
SEQ ID NO: 7 
MKLETLAVHAGYSPDPTTRAVAVPIYQTTSYAFDDTQHGADLFDLKVPGNIYTRIM
NPTNDVLEQRVAALEGGVGALAVASGMAAITYAIQTVAEAGDNIVSVAKLYGGTY
NLLAHTLPRIGIQARFAAHDDVAALEALIDERTKAVFCETIGNPAGNIIDLQALADA
AHRHGVPLIVDNTVATPVLCRPFEHGADIVVHSLTKYMGGHGTSIGGIVVDSGKF
DWAANKSRFPLLNTPDPSYHGVTYTEAFGPAAFIGRCRVVPLRNMGAALSPFNAF
LILQGLETLALRMERHCDNALAVARYLQQHPQVAWVKYAGLADNPEHALARRYL
GGRPAAILSFGIQGGSAAGARFIDALKLVVRLVNIGDAKSLACHPASTTHRQLNAE
ELARAGVSDDMVRLSIGIEHIDDILADLDQALAAAAR
SEQ ID NO: 8 
MSNNIKAYNNVLELIGETPLIKLSQTVASFPGNYYAKAEAFNPGHSSKDRIALHIIE
QAEKKGILKPGDTIIETTSGNTGFSIAMVSRIKGYDCILAVSSKSSADKIDMLKSMG
AKVYYCPANVSADDPRSYYQVAKRLHDEIKNSIYINQYFNDLNTEAHYLTTGPEI
WNQTAGEITHLVACSGTGGTISGTARFLKEQNPNIKILGVDAYGSVLKKYHETQEF
DKDEIYPYRIEGLGKNLIPTATDFDVIDSFTKVKDEDAAHMARKISQTEGLFVGYT
SGAAMQAVKQLVVEGEFDKNSNVVVIFPDHGSRYMSKIYNDQWMQEQGFVDSK
STANEQHIEYIK
SEQ ID NO: 9 
MAVEWTGIANDVTELVGKTPLVYLNKVVDGCVGRIVAKLEFMEPLSSVKDRIGYS
MIADAEEKGLITPGKSVLIEPTSGNTGIGLAFTAAAKGYRLIIVMPASMTLERRMVL
KALGAELVLTDPEKGIRGAFQKVEEIYAKTPNSYVLQQFENPSNPKMHYDTTGPEI
WKATGGKIDALVAGIGTGGTITGSGKFLKEQNPNIKVCAVEPAESPVLVGGQPGSH
GIYGIGAGFIPKVVDVSLLDEVVHVTTSEAIETAKLLAVKEGLFVGVSSGAAAAAA
IKVAKRPEMAGKLIVTIFPSCGERYLSSALFESIREESENMTFEP
SEQ ID NO: 10 
MVFPSPLDFFERGKPTPLVRSRLQLPNGVRVWLKLEWYNPFSLSVKDRPAVEIISRL
SRRVEKGSLVADATSSNFGVALSAVARLYGYRARVYLPGAAEEFGKLLPRLLGAQV
IVDPEAPSTVHLLPRVMKDSKNEGFVHVNQFYNDANFEAHMRGTAREIFVQSRRG
GLALRGVAGSLGTSGHMSAAAFYLQSVDPSIRAVLVQPAQGDSIPGIRRVETGML
WINMLDISYTLAEVTLEEAMEAVVEVARSDGLVIGPSGGAAVKALAKKAAEGDLE
PGDYVVVVPDTGFKYLSLVQNALEGAGDSV
SEQ ID NO: 11 
MGRFILKCLKCGREYSQEYRLTCENDDSFLRAEYLEKKLELRKQPGIGRFHSWLP
VQEELTTEAGPITYKSEALARELGLSNLYIGFSGYWPEKGAFIKTCSFKELEAHPTM
QLLKESGGKAIVLASAGNTGRAFAHVSALTGTDVYIVVPDSGIPKLWLPEEPTDSI
HLISMTPGNDYTDAINLAGRIAKLPGMVPEGGARNVARREGMGTVMLDAAVTIG
KMPDHYFQAVGSGTGGISAWEASLRLREDGRFGSKLPKLQLTQNLPFVPMYNAW
QEGRRDIIPEIDMKDAKKRIEETYATVLTNRAPPYSVTGGLYDALVDTDGIMYAVS
KEEALDAKALFESLEGIDILPPSAVAAASLLKAVEAGNVGKDDTILLNIAGGGFKRL
KEDFTLFQIEPEITVSNPDVPLEELKL
SEQ ID NO: 12 
MTRYDSLLQALGNTPLVGLQRLSPRWDDGRDGPHVRLWAKLEDRNPTGSIKDRPA
VRMIEQAEADGLLRPGATILEPTSGNTGISLAMAARLKGYRLICVMPENTSVERRQ
LLELYGAQIIFSAAEGGSNTAVATAKELAATNPSWVMLYQYGNPANTDSHYCGTGP
ELLADLPEITHFVAGLGTTGTLMGTGRFLREHVANVKIVAAEPRYGEGVYALRNM
DEGFVPELYDPEILTARYSVGAVDAVRRTRELVHTEGIFAGISTGAVLHAALGVGAG
ALAAGERADIALVVADAGWKYLSTGAYAGSLDDAETALEGQLWA
SEQ ID NO: 13 MTVITDITELIGNTPLLRLKNFDVPEGVAVYAKLEMMNPGGSIKDRLGDMLIRDAL 
DSGKVKPGGVIIEATAGNTGIGLALSARKYGLKAIFCVPEHFSREKQQIMQALGASI
IHTPRQDGMQGAIQKAIQLETEIENSYCVLQFKNRVNPSTYYKTLGPEMWEALDG
NIHTFVAGAGSGGTFAGTASFLKEKNPAVKTVIVEPVGSILNGGEPHAHKTEGIGM
EFIPDYMDKSHFDEIYTVTDENAFRLVKEAAEKEGLLIGSSSGAALYAALEEAKKA
SAGTNIVTVFPDSSDRYISKQIYEGGI
SEQ ID NO: 14 
MVQLHQLFPKHEVFAKLEYMNPGGSMKDRPAKYIIEHGIKHGLITENTHLIESTSG
NLGIALAMIAKIKGLKLTCVVDPKISPTNLKIIKSYGANVEMVEEPDAHGGYLMTR
IAKVQELLATIDDAYWINQYANELNWQSHYHGAGTEIVETIKQPIDYFVAPVSTTG
SIMGMSRKIKEVHPNAQIVAVDAKGSVIFGDKPINRELPGIGASRVPEILNRSEINQV
IHVDDYQSALGCRKLIDYEGIFAGGSTGSIIAAIEQLITSIEEGATIVTILPDRGDRYL
DLVYSDTWLEKMKSRQGVKSE
SEQ ID NO: 15 
MTLQYPTIADCVGNTPLVRLQRMAGNTSNTLLLKLEGNNPAGSVKDRPALSMITR
AELRGQIKPGDTLIEATSGNTGIALAMAAAIKGYKMILIMPDNGSAERKAAMTAY
GAELLLVTPEEGMEGARDLAERMAAEGRGQVLDQFANGDNPEAHYTSTGPEIWR
QTQGTITHFVSSMGTTGTIMGNSRYLKEQNPAIQIVGLQPMEGAAIPGIRRWPQEY
LPKIYNAARVDRIIDMAQREAEDTTRRLAREEGIFCGVSSGGAVAGMLRLSAELEN
AVIVAIICDRGDRYLSTGIFDAPN
SEQ ID NO: 16 
MSVFSLKIDIADNKFFNGETSPLFSQSQAKLARQFHQKIAGYRPTPLCALDDLANL
FGVKKILVKDESKRFGLNAFKMLGGAYAIAQLLCEKYHLDIETLSFEHLKNAIGEK
MTFATTTDGNHGRGVAWAAQQLGQNAVIYMPKGSAQERVDAILNLGAECIVTDM
NYDDTVRLTMQHAQQHGWEVVQDTAWEGYTKIPTWIMQGYATLADEAVEQMR
EMGVTPTHVLLQAGVGAMAGGVLGYLVDVYSPQNLHSIIVEPDKADCIYRSGVK
GDIVNVGGDMATIMAGLACGEPNPLGWEILRNCATQFISCQDSVAALGMRVLGNP
YGNDPRIISGESGAVGLGVLAAVHYHPQRQSLMEKLALNKDAVVLVISTEGDTDV
KHYREVVWEGKHAVAP
SEQ ID NO: 17 
MISAFDIFKIGIGPSSSHTVGPMNAGKSFIDRLESSGLLTATSHIVVDLYGSLSLTGK
GHATDVAIIMGLAGNSPQDVVIDEIPAFIELVTRSGRLPVASGAHIVDFPVAKNIIFHP
EMLPRHENGMRITAWKGQEELLSKTYYSVGGGFIVEEEHFGLSHDVETSVPYDFH
SAGELLKMCDYNGLSISGLMMHNELALRSKAEIDAGFARIWQVMHDGIERGMNT
EGVLPGPLNVPRRAVALRRQLVSSDNISNDPMNVIDWINMYALAVSEENAAGGRV
VTAPTNGACGIIPAVLAYYDKFRRPVNERSIARYFLAAGAIGALYKMNASISGAEV
GCQGEIGVACSMAAAGLTELLGGSPAQVCNAAEIAMEHNLGLTCDPVAGQVQIPC
IERNAINAVKAVNAARMAMRRTSAPRVSLDKVIETMYETGKDMNDKYRETSRGG
LAIKVVCG
SEQ ID NO: 18 
MENAKMNSLIAQYPLVKDLVALKETTWFNPGTTSLAEGLPYVGLTEQDVQDAHA
RLSRFAPYLAKAFPETAATGGIIESELVAIPAMQKRLEKEYQQPISGQLLLKKDSHLP
ISGSIKARGGIYEVLAHAEKLALEAGLLTLDDDYSKLLSPEFKQFFSQYSIAVGSTG
NLGLSIGIMSARIGFKVTVHMSADARAWKKAKLRSHGVTVVEYEQDYGVAVEEG
RKAAQSDPNCFFIDDENSRTLFLGYSVAGQRLKAQFAQQGRIVDADNPLFVYLPC
GVGGGPGGVAFGLKLAFGDHVHCFFAEPTHSPCMLLGVHTGLHDQISVQDIGIDN
LTAADGLAVGRASGFVGRAMERLLDGFYTLSDQTMYDMLGWLAQEEGIRLEPSA
LAGMAGPQRVCASVSYQQMHGFSAEQLRNTTHLVWATGGGMVPEEEMNQYLA
KGR
SEQ ID NO: 19 
MSIVRLYTARQLPRYRPLRFVRFNSTQVLSPKQRWTELKDTDFIKDSNGEEQPDYV
KLILTSRVYDVVDDAGSPLTYAINLSHRCGTNVYLKREDLLPVFSFKLRGAYNMIA
 
HLHSNSPTPVPGVIACSAGNHAQGVAFSANKLNIPATIVMPTPTPSIKYTNVSRLGS
QVVLYGDDFDSAKQECARLSAEQNLIDIPPFNHPYVIAGQGTVALEITRQLRLDKL
DAIFVPVGGGGLIAGIAVYLKQIAPHVKIIGVETYDADAMYQSLKNHKSTVLDKV
GLFADGTAVKVLGDETWRLAKDLVDEVVLVTTDELCAAIKDIFEDTRSITEPSGAL
SVAGLKRYIEDHSEIDHKGKTYVPVLSGANMNFDRLRFVSERAVLGEGREVSLAV
TIPEQPGEFAKLQKLINPRAITEFSYRTSGEPTANIFVSFNVIDKKKEKALVINAMTE
AGYKVVDISENELAKSHGRYLVGGKSSIMVMILVKSCVDSISQKDSKKNSNNS
SEQ ID NO: 20 
MTDSVTGAGDGTRAVRAGLPEPVKHEPTLPGPVFAAHFHLPGDPTGPYTYGRDE
NPTWTHLERAIGELEAPGRDDVETLVFASGMAAISAVLFSQLSAGDTVVLPDDGY
QVLPLVRAQLEAYGVEVRTAPTGGDAQLDVLDGAKLLWIETPSNPGLDVCDVRRL
VEAAHARGCLVAVDNTLATPLGQRPLELGADFSVASGTKQLTGHGDILLGYVTGG
DAAAMASVRRWRKIVGAIPGPMEAWLAHRSIATLQLRVDRQNANALVIAEALRD
RHEDLGVRYPGLPGDPSHKIASQQMRRFGCVVSFTLPTRARADRFLDALRLVDDA
TSFGGVRSTAERRGRWGGDAVPEGFIRFSAGAEDPQDLVADVLRALNESSQPTPSE
SEQ ID NO: 21 
MTASSDHPITTPPRAPSPVHGFGTLAVHAGSPHDPATGAVIEAISLSTTFAQTAVGKP
VGEFEYSRSSNPNRANFEKMVAALEHAKYALAYSSGSATTANILQSLAAGSHVISV
SDVYGGTHRYFTQVAKAHGVKVTFTPEIEVDIRDHITDATKLVWIETPSNPTLRLV
DIRAVATAAHERGILVVVDNTFLSPYVQNPLDHGADIVVHSVTKYINGHSDVVMG
VAAFNSDELYARLSFLQNAIGAVPSAFDSWLAHRGAKTLHLRAREATTNATAIAHA
LEASPLVISVNYPGLESHPHRAIALKQHRNGMGGGMLSFRIHGGHAAAEKFCQYT
KIFTLAESLGGVESLCEIPSSMTHAGIPKAQREAVGIFDDLVRISCGVEDAEDLKAD
VLQALERAVADAANGVSNGVNGTH
SEQ ID NO: 22 
MKKKTLMIHGGITGDEKTGAVSVPIYQVSTYKQPKAGQHTGYEYSRTANPTRTAL
EALVTELESGEAGYAFSSGMAAITAVMMLFNSGDHVVLTDDVYGGTYRVMTKVL
NRLGIESTFVDTSSREEVEKAIRPNTKAIYIETPTNPLLKITDLTLMADIAKKAGVLL
IVDNTFNTPYFQQPLTLGADIVLHSATKYLGGHSDVVGGLVVTASKELGEELHFVQ
NSTGGVLGPQDSWLLMRGIKTLGLRMEAIDQNARKIASFLENHPAVQTLYYPGSS
NHPGHELAKTQGAGFGGMISFDIGSEERVDAFLGNLKLFTIAESLGAVESLISVPAR
MTHASIPRERRLELGITDGLIRISVGIEDAEDLLEDIGQALENI
SEQ ID NO: 23 
MATVSSCLLRRSRTASRIFKTSLRCFSTTSSSAQTVSGSSPFPFTGTNIKTNVSQLIGR
TPLVYLSKISEGSGAYIAVKQEMMQPTASVKDRPALAMIEDAEKKGLISPGKTVLIE
PTSGNMGISMAFMAAMKGYKMVLTMPSYTSMERRVVMRAFGADLILTDPDKGM
GGTVKKANQLLDSTPDGFMLQQFNNPANTQVHFETTGPEIWEDTQGKVDIFVMG
IGSGGTVSGVGRYLKSQNPNVKIYGVEPAESNILNGGKPGPHLITGNGVGFKPEIL
DMDVMDAVLEVKSDDAVKMARQLALQEGLLVGISSGANTIAALDLAKRPENKG
KLIVTIHPSFGERYLSSALFKELREEAENMQPVPVE
SEQ ID NO: 24 
MSGIYPAPVRLESHRQQFPALANKAYFNYGGQGTLPQASLEAIQQAYEYIQRHGPF
SAGVNAWIIEEANKTREAIASELGAPPEAIAITEDVTVGCNIAMWGINWQAGDHL
LLTDCEHPSVIATAQEIARRFDVEVSTCPILSTLNQGDPTAVIAQHLRTQTRLVVLSH
ILWNTGQVLPVKEIVEVCRNYSSSSQTIRVLVDAAQSVGCLPLNLTELGADFYAFT
GHKWWCGPEGIGGLYVRPDAWESLNPTFIGWRGIVSNQAGKPTGWKPNAQRFEV
ATSAYPLYAGVRSAIATHQQWGTPQERYQQICQISEYLWQRLSQLDFVKCLRTSPP
QAGLVSFVLTNEHSHQQLVKSLEQQNLMLRTILDPNCVRACVHYFTQHAEIDKLIE
AMKKIKSKG
SEQ ID NO: 25 MRDSHHNTGFSTRAIHHGYDPLSHGGALVPPVYQTATYAFPTVEYGAACFAGEEP 
GHFYSRISNPTLALLEQRMASLEGGEAGLALASGMGAITSTLWTLLRPGDELIVGR
TLYGCTFAFLHHGIGEFGVKVRHVDLNDAKALKAAISSKTRMIYFETPANPNMQL
VDIAAVVEAVRGHDVHVVVDNTYCTPYLQRPLELGADLVVHSATKYLSGHGDIT
AGLVVGRKVLVDRIRLEGLKDMTGAVLSPHDAALLMRGIKTLALRMDRHCANA
QQVAEFLVRQPQVELIHYPGLPSFAQYALAQRQMRLPGGMIAFELKGGIEAGRRF
MNALQLFARAVSLGDAESLAQHPASMTHSSYTPQERAHHGISEGLVRLSVGLEDV
EDLLADVEQALQACC
SEQ ID NO: 26 
MTSLKTKVIHGGISTDRTTGAVSVPIYQTSTYKQNGLGKPKEYEYSRSGNPTRHAL
EELIADLEGGVQGFAFSSGLAGIHAVLSLFSAGDHIILADDVYGGTFRLMDKVLTK
TGIIYDLVDLSNLEDLKAAFKAETKAVYFETPSNPLLKVLDIKEISSIAKAHNALTLV
DNTFATPYLQQPIALGADIVLHSATKYLGGHSDVVAGLVTTNSNELASEIGFLQNSI
GAVLGPQDSWLVQRGIKTLALRMEAHSANAQKIAEFLEASQAVSKVYYPGLVNH
EGHEIAKKQMTAFGGMISFELTDENAVKNFVENLRYFTLAESLGGVESLIEVPAVM
THASIPKELREEIGIKDGLIRLSVGVEALEDLLTDLKEALEKE
SEQ ID NO: 27 
MPLHNLTRFPRLEFIGAPTPLEYLPRFSDYLGREIFIKRDDVTPMAMGGNKLRKLE
FLAADALREGADTLITAGAIQSNHVRQTAAVAAKLGLHCVALLENPIGTTAENYLT
NGNRLLLDLFNTQIEMCDALTDPNAQLEELATRVEAQGFRPYVIPVGGSNALGAL
GYVESALEIAQQCEGAVNISSVVVASGSAGTHAGLAVGLEHLMPESELIGVTVSRS
VADQLPKVVNLQQAIAKELELTASAEILLWDDYFAPGYGVPNDEGMEAVKLLARL
EGILLDPVYTGKAMAGLIDGISQKRFKDEGPILFIHTGGAPALFAYHPHV
SEQ ID NO: 28 
MIFSVDKVRADFPVLSREVNGLPLAYLDSAASAQKPSQVIDAEAEFYRHGYAAVH
RGIHTLSAQATEKMENVRKRASLFINARSAEELVFVRGTTEGINLVANSWGNSNVR
AGDNIIISQMEHHANIVPWQMLCARVGAELRVIPLNPDGTLQLETLPTLFDEKTRL
LAITHVSNVLGTENPLAEMITLAHQHGAKVLVDGAQAVMHHPVDVQALDCDFY
VFSGHKLYGPTGIGILYVKEALLQEMPPWEGGGSMIATVSLSEGTTWTKAPWRFE
AGTPNTGGIIGLGAALEYVSALGLNNIAEYEQNLMHYALSQLESVPDLTLYGPQNR
LGVIAFNLGKHHAYDVGSFLDNYGIAVRTGHHCAMPLMAYYNVPAMCRASLAM
YNTHEEVDRLVTGLQRIHRLLG
SEQ ID NO: 29 
MHLARYPRRFIAHLPTPLERLDRLTAELGGPEIWIKRDDCTGLSTGGNKTRKLEFL
MAEAELQGADMVMTQGATQSNHARQTAAFAAKLGMDCHILLEDRTGSNNANY
NNNGNVLLDHLHGATTEKRPGSGLDMNAEMEKVAEKFRADGRKVYTIPGGGSNP
TGALGYVNCAFEMLNQFNERGLKVDHIVHATGSAGTQAGLITGLQAMNAQIPLL
GIGVRAPKPKQEENVYNLACATAEKLGCPGVVAREDVVANTDYVGEGYGIPTESG
LEAIRMFAELEAILLDPVYSAKGAAGFIDLIRKGHFKKGERVVFLHTGGAVALFGY
DNAFDYSGRWVA
SEQ ID NO: 30 
MDDYKRILITKILKNEVTEALGCTEVGLIGYAVSLCNISDPFSIEKIELTLNNGSFKN
AYAVGVPNTKKYGILPAVVGGLLGDHKNKLLVFNGIKYSQKLEDFIKERLKIRVINS
PLYCGVKIKDNSGNTFESLIKDNHLNVVIPKINNKLISEINGSEKEEYKNLELLDFLE
YIDEIPEEIIQLVEKTIYTNNNLIKGDFLNFGNDCLSNMVNKTTSACNTRMIGENMP
AMSVAKSGNMGIMATLPIIAYDYSNEQNQEKLIKSILLSVLVTIYATYKSSYLSSMC
GCVSKGGMGAVIGLCYYKNGKNIKKLDSAARTFTANLPGIICDGGKVGCALKLAS
GCFAAYSSLFVDISYENGIVGKNFKECVENISEISKIMGDLDSDIVKIMSKKEI
SEQ ID NO: 31

MANDRSVDWKFSGSEAAKEASAASLGTYSSKLFALCDPLGKPILPPRSKSAETSHT
AEKAVVEAVLCGTGNAYAPSLGLPVARSAVAEYLNRDLPKKLTADDVFMTVGGK
QAIELTVDILAKPKANVLLPKPGFPWDVVRTIYKNLEVRKYDFIPEQNYEIDFDSV

RALVDENTFAIFIINPHNPNGNTYSEVHLKKLAELARELRIMVVSDEVFRWTVFGS
NPFVPMGKFSSIVPVVTLGSISKGWIVPGWRTGWVALHDLDGVFKCTKVLTAAKQ
FLVINSKPPTVIQAALPTILENTPEEFFHKRQKFLKEKADFAYSKLKEIPTLTCYLKP
EACTFLWTELNLSRFADIKDDEEFCEKLATEENLVLLPGIAFGLKNWARHSIDMEIS
TLEDALGRLKSFCERHSIIIEVPLKDVNGVK

## Claims

1. A process for preparing L-methionine, comprising steps of:
a) providing a reaction medium, comprising L-homoserine or a salt, an ester, an amide or an anhydride thereof, a methylthio donor, and an enzyme selected from the group consisting of a transferase which transfers alkyl other than methyl or aryl, ammonia lyase, and a lyase which catalyzes the cleavage of a carbon-sulfur bond;
b) incubating the reaction medium to produce a reaction product comprising L-methionine or a salt, an ester, or an amide thereof; and
optionally,
c) recovering the L-methionine or the salt, the ester, or the amide thereof from the reaction medium.

2. The process according to claim 1, wherein
the enzyme is selected from the group consisting of enzymes with the EC numbers of EC 2.5.1.-, EC 4.3.1.- and EC 4.4.1.-.

3. The process according to claim 1 or 2, wherein
the reaction medium further comprises phosphopyridoxal or a salt thereof.

4. The process according to any one of claims 1-3, wherein
the enzyme is selected from the group consisting of enzymes with the EC numbers of EC 2.5.1.47, EC 2.5.1.48, EC 2.5.1.49, EC 2.5.1.51, EC 2.5.1.52, EC 2.5.1.65, EC 2.5.1.76, EC 2.5.1.113, EC 2.5.1.134, EC 2.5.1.140, EC 2.5.1.144, EC 4.3.1.15, EC 4.3.1.17, EC 4.3.1.18, EC 4.3.1.19, EC 4.4.1.1, EC 4.4.1.2, EC 4.4.1.9, EC 4.4.1.10, EC 4.4.1.11, EC 4.4.1.13, EC 4.4.1.15, EC 4.4.1.16, EC 4.4.1.25, EC 4.4.1.28 and EC 4.4.1.35.

5. The process according to any one of claims 1-4, wherein
the enzyme is derived from an organism selected from the group consisting of *Thermus thermophiles, Lysinibacillus sphaericus, Clostridioides difficile, Clostridium novyi, Geobacillus stearothermophilus, Thermotoga maritima, Pseudomonas aeruginosa, Nonlabens dokdonensis, Leucaena leucocephala, Aeropyrum pernix, Methanosarcina acetivorans, Mycobacterium tuberculosis, Bacillus subtilis, Staphylococcus aureus, Pseudomonas fluorescens, Escherichia coli, Candida maltosa, Streptomyces phaeochromogenes, Neurospora crassa, Spinacia oleracea, Cyanobacteria bacterium, Pseudomonas putida, Lactococcus lactis, Ruegeria pomeroyi, Methanococcus maripaludis* and *Camelina sativa.*

6. The process according to any one of claims 1-5, wherein
the enzyme is selected from the group consisting of cysteine synthase derived from *Thermus thermophiles,* cystathionine γ-synthase derived from *Lysinibacillus sphaericus,* O-acetyl homoserine aminocarboxypropyltransferase derived from *Clostridioides difficile,* O-acetyl homoserine aminocarboxypropyltransferase derived from *Clostridium novyi,* O-acetyl homoserine aminocarboxypropyltransferase derived from *Geobacillus stearothermophilus,* O-acetyl homoserine aminocarboxypropyltransferase derived from *Thermotoga maritima,* O-acetyl homoserine aminocarboxypropyltransferase derived from *Pseudomonas aeruginosa,* β pyrazolylalanine synthase derived from *Nonlabens dokdonensis,* L-mimosine synthase derived from *Leucaena leucocephala,* O-phosphoserine sulfhydrylase derived from *Aeropyrum pernix,* cysteine synthase derived from *Methanosarcina acetivorans,* [CysO sulfur-carrier protein]-thiocarboxylate-dependent cysteine synthase derived from *Mycobacterium tuberculosis,* cystathionine β synthase derived from *Bacillus subtilis,* N-(2-amino-2-carboxyethyl)-L-glutamate synthase derived from *Staphylococcus aureus,* S-sulfo-L-cysteine synthase derived from *Pseudomonas fluorescens,* diaminopropionate ammonia-lyase derived from *Escherichia coli,* L-serine ammonia-lyase derived from *Escherichia coli,* D-serine ammonia-lyase derived from *Escherichia coli,* threonine ammonia-lyase derived from *Candida maltosa,* cystathionine γ lyase derived from *Streptomyces phaeochromogenes,* cystathionine γ lyase derived from *Neurospora crassa,* homocysteine desulfhydrase derived from *Bacillus subtilis,* L-3-cyanoalanine synthase derived from *Spinacia oleracea,* cysteine lyase derived from *Cyanobacteria bacterium,* methionine γ lyase derived from *Pseudomonas putida,* cysteine-S-conjugate β lyase derived from *Lactococcus lactis,* D-cysteine desulfhydrase derived from *Escherichia coli,* selenocysteine lyase derived from *Escherichia coli,* L-cysteate sulfo-lyase derived from *Ruegeria pomeroyi,* L-cysteine desulfidase derived from *Methanococcus maripaludis* and L-cystine β lyase derived from *Camelina sativa.*

7. The process according to any one of claims 1-6, wherein
the enzyme comprises an amino acid sequence selected from the group consisting of SEQ ID NO: 1-31.

8. The process according to any one of claims 1-7, wherein
the process is a cell-free process.

9. The process according to any one of claims 1-7, wherein
the reaction medium in step a) comprises a host cell expressing the enzyme.

10. The process according to claim 9, wherein
the process further comprises a step of culturing the host cell.

11. The process according to any one of claims 1-10, wherein
in step b), the reaction medium is incubated at 30-40°C, 32-39°C, 34-38°C, 36-38°C or 37°C.
